Europäisches Patentamt

European Patent Office

Office européen des brevets

(11) Veröffentlichungsnummer: **0 218 879**
**B1**

# (12) EUROPÄISCHE PATENTSCHRIFT

(45) Veröffentlichungstag der Patentschrift:
21.06.89

(21) Anmeldenummer: 86112089.7

(22) Anmeldetag: 02.09.86

(51) Int. Cl.⁴: **C 07 C 43/295,** C 07 C 41/34, C 07 C 41/09

(54) Verfahren zur Herstellung von 4,4'-Dihydroxydiphenylether.

(30) Priorität: 14.09.85 DE 3532881

(43) Veröffentlichungstag der Anmeldung:
22.04.87 Patentblatt 87/17

(45) Bekanntmachung des Hinweises auf die Patenterteilung:
21.06.89 Patentblatt 89/25

(84) Benannte Vertragsstaaten:
BE CH DE FR GB IT LI NL

(56) Entgegenhaltungen:
FR-A-2 194 676
US-A-4 306 094

PATENTS ABSTRACTS OF JAPAN, Band 4, Nr. 190
(C-37) 672 , 26. Dezember 1980; & JP-A-55 129 237 (UBE
KOSAN K.K.) 06-10-1980

(73) Patentinhaber: HOECHST AKTIENGESELLSCHAFT,
Postfach 80 03 20, D-6230 Frankfurt am Main 80
(DE)

(72) Erfinder: Vorwerk, Edgar, Dr., Geisenheimer
Strasse 88, D-6000 Frankfurt am Main 71 (DE)

## Beschreibung

Die Erfindung betrifft ein Verfahren zur Herstellung von 4,4'-Dihydroxydiphenylether durch Dehydratisierung von Hydrochinon mit Sulfonsäuren oder Schwefelsäure als Katalysatoren bei erhöhten Temperaturen.

Die Herstellung von 4,4'-Dihydroxydiphenylether aus Hydrochinon unter dehydratisierenden Bedingungen ist bereits mehrfach beschrieben worden.

Nach der US-PS 2 739 171 wird die Dehydratisierung von Hydrochinon bevorzugt in Gegenwart von 95 - 100 %-iger Flußsäure durchgeführt, aber auch Phosphorsäure, Calciumphosphate sowie sauer reagierende Oxide oder Oxidmischungen des Siliciums, Aluminiums, Zirkoniums und Magnesiums werden als Dehydratisierungsmittel genannt. Nachteilig sind die Umsätze von nur 30 % mit einer nur 47 %-igen Selektivität zum 4,4'-Dihydroxydiphenylether. Eine Methode zur Abtrennung des Ethers von den Nebenprodukten wird nicht angegeben.

In der deutschen Offenlegungsschrift 2 237 762 wird die Verwendung von mit Säuren aktivierten natürlichen oder synthetischen Aluminiumsilikaten (Montmorillonit, Bentonit) zur Wasserabspaltung aus Hydrochinon beschrieben. Zur Anwendung kommt eine relativ hohe Katalysatormenge, die zwischen 10 und 50 Gew.-%, bezogen auf das eingesetzte Hydrochinon, liegt. Aus dem anfallenden Reaktionsgemisch wird der 4,4'-Dihydroxydiphenylether in aufwendiger Weise von unumgesetztem Hydrochinon und höheren Kondensationsprodukten durch Codestillation mit geeigneten, hochsiedenden Lösungsmitteln abgetrennt.

Schließlich ist aus der Japanischen Auslegeschrift SHO-55-129 237 bekannt, daß sich der 4,4'-Dihydroxydiphenylether aus Hydrochinon und Schwefelsäure oder Sulfonsäuren bei erhöhter Temperatur herstellen läßt. Die Abtrennung des gewünschten Ethers vom Katalysator, unumgesetztem Hydrochinon und höheren Kondensationsprodukten erfolgt durch Extraktion der gesamten phenolischen Bestandteile mit einem geeigneten organischen Lösungsmittel, Entfernung dieses Lösungsmittels und anschließender Destillation des Rückstandes.

Diese Verfahren liefern also den 4,4'-Dihydroxydiphenylether im Gemisch mit unumgesetztem Hydrochinon und höher kondensierten Produkten, deren Abtrennung aufwendig und technisch bisher wenig befriedigend ist.

Weiter ist aus der US-Patentschrift 4 306 094 die Dehydratisierung von Hydrochinon mit Schwefelsäure, Ethylsulfonsäure oder p-Toluolsulfonsäure in Xylol bei 140°C bekannt. Aus dem dehydratisierten Reaktionsgemisch wird mit warmem Wasser das Hydrochinon herausgewaschen. Das zurückbleibende Gemisch aus gewünschtem Diphenylether und unerwünschtem Triphenylether muß anschließend aus einem Lösungsmittel umkristallisiert werden, um den Diphenylether rein zu erhalten. Wie die Beispiele der Literaturstelle zeigen, ist dies aber anscheinend nur begrenzt gelungen.

Bei diesem Verfahren ist die Gegenwart eines Lösungsmittels zwingend vorgeschrieben, insbesondere anscheinend, um die Reaktionstemperatur relativ niedrig halten zu können, nämlich bei 80 - 180°C, was für sehr wichtig gehalten wird.

Überraschungsweise wurde nun gefunden, daß in Abwesenheit eines Lösungsmittels und bei höherer Temperatur (170 - 250°C) mit Erfolg gearbeitet werden kann. Und dabei wird ein reines Produkt in einem Reinigungsschritt erhalten. Dieser besteht darin, daß das dehydratisierte Reaktionsgemisch in einem Überschuß von heißem Wasser verrührt wird, so daß man anschließend durch einfaches Abkühlen der entstandenen Lösung den Diphenylether selektiv auskristallisieren lassen kann, wobei das Hydrochinon in Lösung bleibt.

Gegenstand der Erfindung ist demgemäß ein Verfahren zur Herstellung vom 4,4'-Dihydroxydiphenylether durch Dehydratisierung von Hydrochinon mit Sulfonsäuren oder Schwefelsäure als Katalysatoren bei erhöhten Temperaturen, dadurch gekennzeichnet, daß man die Dehydratisierung in der Schmelze bei einer Temperatur von 170 bis 250°C durchführt, das Reaktionswasser austreibt oder entzieht, dann das noch heiße und flüssige Reaktionsgemisch bei 80 - 110°C mit einem Überschuß Wasser verrührt, die nicht gelösten, höher kondensierten Nebenprodukte abtrennt und aus dem Filtrat den Ether auskristallisieren läßt.

Als Katalysatoren benutzt man vorzugsweise aromatische Sulfonsäuren wie z. B. p-Toluolsulfonsäure, Benzolsulfonsäure, Chlorbenzolsulfonsäure, Naphthalin-1-sulfonsäure, Naphthalin-2-sulfonsäure, Hydroxynaphthalinsulfonsäuren, 4-Hydroxynaphthalin-2,7-disulfonsäure.

Die eingesetzte Katalysatormenge beträgt vorzugsweise 1 - 10 Gew.-%, bezogen auf das Hydrochinon.

Die Dehydratisierung wird in der Schmelze durchgeführt, und zwar bei Temperaturen von 170 - 250°C, insbesondere 200 - 230°C. Gerechnet vom Beginn des Vorliegens einer vollständigen Schmelze an, beträgt die Reaktionsdauer im allgemeinen einige Minuten bis wenige Stunden, vorzugsweise 30 Minuten bis 1,5 Stunden.

Das entstehende Reaktionswasser wird vorzugsweise durch einen Inertgasstrom, beispielsweise Stickstoff, Helium oder Kohlendioxid, ausgetrieben. Man kann aber auch das Reaktionswasser über einen absteigenden Kühler dem Reaktionsgemisch entziehen, wobei man kein Inertgas benötigt. Zur Abtrennung des Reaktionsproduktes von unumgesetztem Hydrochinon, höher kondensierten Nebenprodukten und dem Katalysator wird das noch heiße und flüssige Reaktionsgemisch in Wasser eingerührt und auf mindestens 80°C erhitzt. Die Obergrenze für die Temperatur wird natürlich vom Siedepunkt dieser Mischung bestimmt, die im allgemeinen bei etwa 110°C liegt. Die Wassermenge beträgt im allgemeinen das 5 bis 50-fache der Gewichtsmenge des Hydrochinons, bevorzugt das 8 - 20-fache.

Der Zusatz von Aktivkohle kann vorteilhaft sein, ist aber nicht notwendig.

Ungelöst bleiben bei der Wasserbehandlung die höher kondensierten Nebenprodukte, die abgetrennt werden, etwa durch Filtrieren oder Zentrifugieren.

Durch Abkühlung des Filtrats oder Zentrifugats, vorzugsweise auf Temperaturen von 0°C - 40°C, scheidet sich der 4,4'-Dihydroxydiphenylether aus und wird durch Filtration oder Zentrifugieren isoliert.

Aus dem Filtrat oder Zentrifugat dieser Kristallisation kann durch Einengen unumgesetztes Hydrochinon gewonnen werden, das zurückgeführt werden kann.

Das Verfahren kann kontinuierlich oder diskontinuierlich durchgeführt werden.

Der 4,4'-Dihydroxydiphenylether ist eine interessante, bifunktionelle Komponente bei Polykondensationen und Polyadditionen. Die dabei entstehenden Endprodukte können als Isolationsmaterial oder als temperaturbeständige Polymere benutzt werden (DE-PS-1 236 197, US-PS-3 375 297, US-PS-3 236 809).

## Beispiel

In einem Reaktionsgefäß wurden 165,2 g (1,5 Mol) Hydrochinon und 5 g p-Toluolsulfonsäure-Monohydrat gemischt und in ein auf 210°C vorgeheiztes Ölbad gehalten. Nachdem das Reaktionsgemisch vollständig geschmolzen war, wurde für weitere 45 min auf 210°C erhitzt. Das bei der Reaktion freiwerdende Reaktionswasser entfernte man durch einen Stickstoffstrom über einen absteigenden Kühler.

Das noch heiße, flüssige Reaktionsgemisch wurde in 2,5 l Wasser eingerührt und anschließend 45 min zum Sieden erhitzt, wobei man 8 g Aktivkohle zusetzte. Man filtrierte heiß und trennte auf diese Weise von den unlöslichen, höher kondensierten Bestandteilen des Reaktionsgemisches ab, wobei die Filtration durch Verwendung von Filterhilfe vereinfacht wurde. Durch Abkühlung des Filtrats auf 15°C kristallisierte der 4,4'-Dihydroxydiphenylether aus und wurde abgesaugt und isoliert.

Man erhielt 39,0 g 4,4'-Dihydroxydiphenylether, bzw. 36,8 g nach Umkristallisation aus Xylol mit einem Festpunkt von 163 - 165°C (Ausbeute: 40,7 %, bezogen auf umgesetztes Hydrochinon).

Die Mutterlauge wurde eingeengt. Der Rückstand wurde in Aceton aufgenommen und mit ca. 8 g Aktivkohle aufgekocht. 66,64 g Hydrochinon wurden zurückgewonnen, verunreinigt mit ca. 2 % 4,4'-Dihydroxydiphenylether.

## Patentansprüche

1. Verfahren zur Herstellung vom 4,4'-Dihydroxydiphenylether durch Dehydratisierung von Hydrochinon mit Sulfonsäuren oder Schwefelsäure als Katalysatoren bei erhöhten Temperaturen, dadurch gekennzeichnet, daß man die Dehydratisierung in der Schmelze bei einer Temperatur von 170 bis 250°C durchführt, das Reaktionswasser austreibt oder entzieht, dann das noch heiße und flüssige Reaktionsgemisch bei 80 - 110°C mit einem Überschuß Wasser verrührt, die nicht gelösten, höher kondensierten Nebenprodukte abtrennt und aus dem Filtrat den Ether auskristallisieren läßt.

2. Verfahren nach Anspruch 1, dadurch gekennzeichnet, daß die Wassermenge das 5- bis 50-fache der eingesetzten Hydrochinonmenge beträgt.

3. Verfahren nach Anspruch 1, dadurch gekennzeichnet, daß die Wassermenge das 8- bis 20-fache der eingesetzten Hydrochinonmenge beträgt.

## Claims

1. A process for the preparation of 4,4'-dihydroxydiphenyl ether by dehydration of hydroquinone using sulfonic acids or sulfuric acid as catalysts at elevated temperatures, wherein the dehydration is carried out in the melt at a temperature of from 170 to 250°C, the water of reaction is expelled or removed, the reaction mixture, which is still hot and liquid, is stirred with an excess of water at 80 - 110°C, the undissolved, higher condensed byproducts are separated off, and the ether is allowed to crystallize out of the filtrate.

2. The process as claimed in claim 1, wherein the amount of water is 5 to 50 times the amount of hydroquinone employed.

3. The process as claimed in claim 1, wherein the amount of water is 8 to 20 times the amount of hydroquinone employed.

## Revendications

1. Procédé pour préparer l'oxyde de bis-(hydroxy-4 phényle) par déshydratation de l'hydroquinone au moyen d'acides sulfoniques ou d'acide sulfurique comme catalyseurs, à température élevée, procédé caractérisé en ce qu'on effectue la déshydratation sur le mélange fondu, à une température de 170 à 250°C, on entraîne ou élimine l'eau engendrée par la réaction, puis on agite le mélange réactionnel encore chaud et liquide, à 80 - 110°C, avec un excès d'eau, on sépare les produits secondaires à plus haut degré de condensation, qui ne sont pas dissous, et on fait cristalliser l'oxide dans le filtrat.

2. Procédé selon la revendication 1 caractérisé en ce que la quantité d'eau représente de 5 à 50 fois la quantité d'hydroquinone mise en jeu.

3. Procédé selon la revendication 1 caractérisé en ce que la quantité d'eau représente de 8 à 20 fois la quantité d'hydroquinone mise en jeu.